# EUROPEAN PATENT SPECIFICATION

(11) **EP 3 496 673 B1**
(45) Date of publication and mention of the grant of the patent: **30.12.2020**
(21) Application number: 17754942.5
(22) Date of filing: 09.08.2017
(51) Int. Cl.: A61F 5/01

(54) **ORTHOPEDIC DEVICE**
ORTHOPÄDISCHE VORRICHTUNG
DISPOSITIF ORTHOPÉDIQUE

(30) Priority: 09.08.2016 US 201662372367 P
(43) Date of publication of application: 19.06.2019
(73) Proprietor: Össur Iceland EHF, 110 Reykjavik (IS)
(72) Inventor: INGIMUNDARSON, Arni Thor, 110 Reykjavik (IS); SIGURDSSON, Sindri Pall, 110 Reykjavik (IS)
(74) Representative: Klunker IP Patentanwälte PartG mbB
(86) International application number: PCT/US2017/046028
(87) International publication number: WO 2018/031618

(56) References cited:
- US-A- 6 059 834
- US-A1- 2003 114 782
- US-A1- 2013 053 743
- US-A1- 2014 303 534
- US-A1- 2016 081 835

## Description

### FIELD OF ART

The embodiments of this disclosure are directed to an orthopedic device arranged for intimately fitting to, immobilizing, restricting, supporting, and guiding anatomical segments or joints.

### BACKGROUND

Orthopedic braces and supports are arranged to restrict, inhibit, immobilize, or otherwise control motion about anatomical segments or joints of the human body. These braces and supports provide compression, support, and stability. Many known braces and supports incorporate rigid members and hinges for immobilizing a joint or facilitating movement of the joint. Orthopedic bracing has tended toward greater rigidity to provide ultimate immobilization with a reduced margin of error in mobility, thus many device designs are over-engineered.

Document US 2016/081835 A1 relates to a proprioceptive orthosis intended to support a joint such as the knee or elbow of a human body.

Document US 2003/114782 A1 relates to orthopedic supports and, more specifically, to a composite material for use in making elastic compression braces having improved compression support, body heat retention, and breathability during use.

Document US 6 059 834 A is concerned with improved sleeve assemblies useful for orthopedic or prosthetic suspension purposes, which includes anti-slip material applied to a portion of inner surfaces thereof in order to minimize migration or slippage of the sleeve assemblies in use.

There tends to be an inverse relationship between the rigidity of the device and patient comfort/compliance. The more rigid the device is, the less likely it will be worn, especially over extended periods of time. If the patient removes a required device to increase comfort, rehabilitation may be hampered and risk of further injury may be elevated.

Flexible braces and supports exist which offer compression, support, and stability. However, many are formed from synthetic materials such as Neoprene. These types of braces can give rise to allergic reactions and get hot. Because they typically rely on a sheet of homogeneous material, they lack areas having different properties, and may be ill-fitting or ineffective as a joint requires different areas of compression. Orthopedic devices, such as flexible orthopedic devices and sleeves, provide anatomical fit by conforming to a user's anatomy for physiologically correct support. They are flexible and arranged for contouring to a body or joint to minimize movement restriction and discomfort. The devices may be configured to stretch in different ways to enable greater muscle stability. In view of their sizing and fit, these devices not only provide support but also improve circulation, and reduce pain and inflammation. Other types of flexible orthopedic devices and/or sleeves may include neoprene and/or other textiles and materials, and may include other tubular configurations or other shapes and configurations as well.

Unfortunately, known orthopedic devices are often lacking in adequate strapping and instead rely on their elasticity for being maintained on a limb and/or preventing unwanted migration of the device. These devices are limited to uniform elasticity, whereas the user may require different degrees of elasticity depending on where the device is intended be placed over the anatomy, for different types of recoveries from different types of pathologies, and/or for being retained on the user and treatment of specific anatomy. Such devices may not provide sufficient support, and rather offer more proprioceptive than functional capabilities.

In view of the tubular nature of many orthopedic devices including flexible orthopedic devices or sleeves, an issue often arises in migration control. Taking for instance a knee support, as the leg is conical, it is difficult to control the migration of the knee support during repeated movement between flexion and extension. Often straps are used to hold the support on the leg, or light frictional material is applied on the interior of the support. The straps may exert too much compressive force on the leg, creating discomfort and impeding activities of the user.

The frictional material which serves to hold the orthopedic device in place must have low frictional qualities so as to allow the user to slide the support on the leg, which has the undesired effect of reducing effectiveness of anti-migration means. Compensating for the use of low friction materials with a more highly compressive body panel in order to better hold the device in place also creates problems, as highly compressive body panels are extremely difficult to don, and when donned are uncomfortable for a user and are thus not suitable for extended use. These current means for migration control require improvement to balance and optimize migration control, ease of use, and donning and doffing of the support.

Strap designs in existing devices mistake the proper placement of straps and thus do not offer optimal support for patients suffering from pathologies such as osteoarthritis. Many orthopedic devices feature straps that are connected to each other at a crossing or intersecting point on the orthopedic device, usually directly at and over the hinge located on a medial or lateral side of the device. However, this is not an ideal placement of the intersection of the straps, as it does not effectively unload the knee, and thus does not effectively treat the underlying condition, such as osteoarthritis. Straps may also be difficult to repeatedly apply and adjust. Straps may stray from their intended location on the brace, reducing their effectiveness; straps may also get tangled with each other or be damaged by external forces.

Another issue that arises in orthopedic devices is difficulty in donning and doffing due to poor grip on the device by a user. Gripping the tubular body directly can lead to undesired stretching and damage to the device, while also being difficult for a user. Certain orthopedic devices allow for a tab that guides a strap on an outside surface of the device to serve additionally as a "pull tab," but this needs improvement as it can lead to damage of the tab that must also guide a strap, and may also be inconvenient due to its location, as the tab could catch on objects, further causing damage and inconvenience and making long term use difficult.

Orthopedic devices often comprise a patella support for maintaining the patella in position to prevent dislocation. However, patella supports are frequently ineffective at holding the patella in position as the patella supports themselves, which often take the form of a pad attachable within a tubular body, are prone to migration or translation along a user's skin. Patella supports also can create pressure points or be inflexible which further adds to user discomfort.

From the foregoing, there is a need for an orthopedic device that provides improved migration control, comfort, and functionality, while being easier to don/doff, and providing enhanced biomechanical support of a joint, such as by properly locating straps and providing effective patella support.

### SUMMARY

The present invention is defined in claim 1. An orthopedic device according to the disclosure arranged for intimately fitting the limb and joint of the user solves the problem of rigid braces and supports deterring a user from proper use and not facilitating joint motion activities. The orthopedic device also solves the problem of orthopedic devices, including flexible orthopedic devices and sleeves, having ineffective migration control and being difficult to don/doff. The orthopedic device also solves the problem of straps intersecting at a location that is ineffective for treating pathologies such as osteoarthritis, particularly of the knee. The orthopedic device also solves the problem of straps being cumbersome to use due to straying from intended locations, tangling, or being damaged by external forces. The orthopedic device also solves the problem of patella supports being ineffective due to undesired translation along skin, being inflexible, or not offering support.

The orthopedic device may have restrictive cables, straps, and/or restrictive bands or regions integrated and/or attachable into the construction of the device to restrict and guide the joint. These restrictive cables, straps and/or restrictive bands may be formed from metallic yarns, fusible, textiles, thermoplastic fibers, elastics, or other suitable materials and elements. They may be knit or inlayed during construction of the fabric brace, or mounted externally of the brace; alternatively, they may be modular and attachable to the device or components thereof for each adaptation in initial installation and use. In an embodiment, the orthopedic device comprises straps that intersect at a location that is effective for treating pathologies such as osteoarthritis. The orthopedic device may further comprise tabs for assistance in donning/doffing the device.

The embodiments of the orthopedic device define a compliant yet comfortable garment, such as a sleeve or other article, arranged to biomechanically provide motion restriction/facilitation of a joint or body segment. The embodiments are preferably adjustable in functional capacity to match the biomechanical requirements of a user's treatment plan throughout rehabilitation. The orthopedic device includes various comfort factors to facilitate maximal compliance of the user over the duration of their treatment such as profile, breathability, compression, flexibility, and rigidity.

In certain embodiments, the orthopedic device comprises a patella pad with enhanced anti-translation properties that offers support to a patella region and overcomes the problems of existing patella supports by offering enhanced flexibility, pressure relief, and support areas.

Embodiments are provided to offer improved migration control over known devices. Embodiments may include means for removing or mitigating migration control during donning and doffing, while offering improved migration control during use.

In an exemplary embodiment, the migration control comprises a frictional material placed about the orthopedic device or in sections at a predetermined location of the orthopedic device, such as a top or proximal end The frictional material is preferably deposited along an inner surface of the orthopedic device. The support is arranged with a cuff that bears the frictional material, and can be folded over the orthopedic device during donning and doffing to disengage the frictional material from the body of the user. Upon a desired location of the orthopedic device on the user, the cuff can be reverted to a position allowing for engagement of the frictional material to the body of the user.

In these and other possible embodiments, the problems of orthopedic devices including flexible orthopedic devices and sleeves being ineffective at migration control and/or difficult to don/doff, offering ineffective patella support, and being ineffective at mitigating pathologies such as osteoarthritis, are addressed and mitigated.

### BRIEF DESCRIPTION OF THE DRAWINGS

These and other features, aspects, and advantages of the present disclosure will become better understood regarding the following description, appended claims, and accompanying drawings Embodiments of the invention are illustrated only in Figs. 4A-4E.
Fig. 1A is a front elevational view of an example of a knee support according to an orthopedic device.
Fig. 1B is a side elevational view of the example of Fig. 1.
Fig. 2A is a detail view of a material structure in an example of an orthopedic device.
Fig. 2B is a detail view of another material structure in an example of an orthopedic device.
Fig. 2C is a perspective view of another material structure in an example of an orthopedic device.
Fig. 2D is a detail view of the detail IID when the orthopedic device is in extension.
Fig. 2E is a detail view of the detail IID when the orthopedic device is in flexion.
Fig. 3A is a side elevational view of another knee support according to an example of an orthopedic device in a disengaged configuration.
Fig. 3B is a front elevational view of the example of Fig. 3A in an engaged configuration.
Fig. 4A is a side elevational view of another knee support according to an embodiment of an orthopedic device in a disengaged configuration.
Fig. 4B is a front elevational view of the embodiment of Fig. 3A in an engaged configuration.
Fig. 4C is a schematic view of the embodiment of Fig. 4A showing a variation of an upper cuff in a disengaged configuration.
Fig. 4D is a schematic view showing a frictional material pattern.
Fig. 4E is a schematic view of a variation of a frictional material pattern.
Fig. 5 is a front elevational view of another example of a knee support according to an example of an orthopedic device.
Fig. 6A is a front elevational view of another example of a knee support according to an example of an orthopedic device.
Fig. 6B is a side elevational view of the example of Fig. 6A.
Fig. 7A is a perspective view of another example of a knee support according to an example of an orthopedic device.
Fig. 7B is a perspective view of a variation of the knee support of Fig. 7A.
Fig. 8A is a plan view of a knee support according to an example of an orthopedic device in an open configuration.
Fig. 8B is a plan view of a patella buttress for use in the aforementioned examples of knee supports according to an example of an orthopedic device.
Fig. 8C is an elevational view of a stay for use in the aforementioned examples of knee supports according to an orthopedic device.
Fig. 9 is an elevational view of pull tabs according to an example of an orthopedic device.
Fig. 10 is an elevational view of pull tabs according to another example of an orthopedic device.

### DETAILED DESCRIPTION OF VARIOUS EMBODIMENTS

### A. Fabrication of Various Embodiments

Various embodiments of the orthopedic device may utilize flat knitting, which allows production of textile structures into a final desired shape so there is no cutting and very minimal waste. Flat knitted elements are formed directly in the desired three-dimensional shapes or modular panels, which can help avoid the need to use additional support structures.

In engineering the stitches to stretch, restrict, pad, or contour to body shapes, using multiple types of yarns and/or combined stitch patterns at strategic placements, several desired performance characteristics can be localized into performance zones within a single, unitary construction. Various pockets, channels, and tunnels can be formed by the knitting to introduce: restrictive stays, pads, hot/cold packs, hyper elastic materials, inflatable pouches (liquid or air), webbing, hardware, and/or other customizable elements of bracing and support. Inflatable areas in the knit pouches can exert pressure and force on the desired areas and are customized to a patient's condition.

Embodiments of the functional knit orthopedic device preferably form a knitted textile support having a shape created on a knitting loom including, but not limited to, various warp knitting, circular knitting, or weft (flat) knitting processes.

Embodiments of the functional knit orthopedic device may take the form of a garment. Support areas may include several textile elements combined into one textile support panel with unitary construction or a group of modular panels for treating a medical indication. The body of the textile panel or panels provides for biomechanical range of motion, compression, and therapeutic elements integrated into a single panel, or panels that may form a garment. Three-dimensional flat knitting allows production of these textile structures into a final desired shape to avoid cutting and waste.

Each panel or series of panels may have areas of gradient levels of stretch, flexure, rigidity, and restrictive elements integrated into the fabric by mapping the levels of motion or restriction required for a medical indication. This is achieved through a corresponding system of knit stitching techniques that start with yarns. Knit stitching techniques create varying degrees of elasticity, rigidity, open channels, tunnels, and intarsia zones of specialized yarns integrated into the base fabric. The first area of knitting may be formed of a first stitch configuration, and the second area may be formed of a second stitch configuration different from the first stitch configuration to impart varying textures or properties to a surface of the textile element. These properties may include anti-bacterial, cooling, warming, elasticity, rigidity, compression, wicking, and/or color.

The knitted base of the device can comprise natural and/or synthetic yarns: silk, wool, polyester, nylon, olefin, and interlaced with specialty yarns: moisture management, elasticized, fusible, metallic, Kevlar, silicone, and other types of performance yarns knit into fully fashioned, textured, intarsia, or three-dimensional regions and appendages such as connected tubes, circles, open cuboids, straps, spheres, and other integrated knit shapes.

Flat knitted elements may be formed directly in the desired three-dimensional shapes or modular panels, which can help avoid the need to use additional support structures and emerge from the machine ready to be sewn together, advantageously saving on manufacturing costs. This fully fashioned knitting technique adds or drops stitches to create custom two- and three-dimensional shapes appropriate to the desired finished garment structure.

The intarsia areas may comprise threads or yarns, which are isolated into specialized zones, using silicon, Kevlar, fusible, nylon monofilament, Dynema, spandex, and/or other specialty performance yarns knit into the fabric to enable that region to perform a function. These areas can be arranged into any flat, textured, or three-dimensional shape required for load mapping the garment for the medical indication.

The load mapped zones may be further achieved by combining knit structures (knit, tuck, miss), transferring loops, dropping or adding needles, segmenting takedown in varying rates across the width of the garment fabric, varying structural elements, inlaying yarns, weft insertion, direct feed, warp insertion, and varying speed of yarns fed into the system.

Besides standard knitting feeders, several types of specialized knitting feeders may isolate, apply, and integrate these yarns into the base fabric of this garment: intarsia feeders, in-lay, direct feeders, and plaiting feeders. With the plaiting, the yarn may lie in the isolated area but only on the face or the back of the fabric.

### B. Biomechanics of Various Embodiments

Control of joint range of motion includes motion inhibition, restriction, or prevention. This may be obtained through altering tension in a garment on a tangent to the skin controlling motion through shear loads (at a tangent to the skin). Control of joint range of motion and soft tissue may be assisted through compression or loading, at or near a normal direction to the surface of the skin and/or garment.

In embodiments of the orthopedic device, normal motion may be disrupted by an intimately fitting garment with restrictive bands integrated therein for constructing the functional knit orthopedic device. The restriction may be created through altering both the yarn and the weave of the fabric. The interface between fabric and skin can vary. Some areas can be low stretch and have high friction or tackiness bonding the fabric to the skin. These regions form an anchor for stabilization of the garment to the core and the extremity.

Other textile areas can have high stretch and offer low friction, allowing the body segment to move freely. By carefully selecting the anchor zones and orienting restrictive bands within the textile, embodiments can provide restriction or guidance to the joint in question, preventing injurious movement and even encouraging safe motion strategies about the joint.

### C. Definitions

Numerous orthopedic device embodiments and components for use therewith are described herein, with particular focus given to flexible orthopedic devices and/or sleeves, and components directed to joints, in particular the knee joint and surrounding areas. The orthopedic device embodiments may serve in protective, preventative, or remedial capacities. While the orthopedic device is described within the context of a preferred embodiment that is directed to the knee, many of the features described herein may be extended to various orthopedic devices and components that secure other joints and body parts. Other flexible orthopedic devices and sleeves may comprise sleeves and devices comprising neoprene and/or other textiles and materials, and may include other tubular configurations or other shapes and configurations as well.

The orthopedic device embodiments and components for use therewith may be dimensioned to accommodate different types, shapes, and sizes of human joints and appendages. In addition, embodiments may be modified to orient principal forces exerted by strap systems of the embodiments at any desirable location to secure the device onto a leg in order to stabilize the joint.

The knee joint comprises two joints, lateral and medial, between the femur and tibia, and one arthrodial joint between the patella and femur. The primary movements of the knee comprise flexion, i.e., rearward rotational movement of the tibia relative to the femur (the completion of flexion ideally resulting in a fully bent leg), and extension, i.e., forward rotational movement of the tibia relative to the femur (the completion of extension ideally resulting in a fully straightened leg).

For explanatory purposes, each orthopedic device embodiment or component thereof described herein may be divided into sections which are denoted by general anatomical terms for the human body. Such anatomical terms are provided to distinguish various elements of the device embodiments from one another, but which are not to be considered to limit the scope of the disclosure.

Each of these terms may be used in reference to a human leg, by way of example, which is divided in similar sections with a proximal-distal plane generally extending along the meniscus of the knee between the femur and tibia. The terms "proximal" and "distal" generally refer to locations of the device that correspond to the location of the leg relative to the point of attachment of the leg to the body. The terms "upper" and "lower" may be used in combination with "proximal" and "distal" to connote gradations in location of "proximal" and "distal." The location where the device corresponds to the knee joint is used herein to generally delimit the proximal and distal sections of the device.

The embodiments of the orthopedic device can also be considered to fall within "anterior" and "posterior" sections of an anterior-posterior plane. The anterior-posterior plane generally corresponds to the coronal or frontal plane of a human leg which lies along the central longitudinal axis of a body. A posterior side or element is therefore located behind this anterior-posterior plane, whereas an anterior side or element is located in front of the anterior-posterior plane.

The terms "inwardly" or "inner" commonly used herein to distinguish the side of the device that may be directed to the posterior side of the device and specifically adjacent to the leg of the wearer of the device. Contrariwise, the term "outwardly" or "outer" are used to denote the side of the device that is opposite to the inwardly side.

The terms "medial" and "lateral" are relative terms that are generally understood as indicating location with respect to the midsaggital plane or midline. Therefore, elements that are located near the midline are referred to as "medial" and those elements that are further from the midline are considered to be "lateral." The term "central" is used to denote the area along the midline of a joint thereby dividing and sharing regions of the medial and lateral regions.

The terms "rigid" and "flexible" may distinguish characteristics of portions of certain features of the orthopedic device. The term "rigid" should denote an element of the device is devoid of flexibility. Within the context of frame or support members or shells that are "rigid," it should indicate that they do not lose their overall shape when force is applied, and they may break if bent with sufficient force. The term "flexible" should denote that features are capable of repeated bending such that the features may be bent into retained shapes or the features retain no general shape, but continuously deform when force is applied.

### D. Detailed Description of Various Embodiments

In any of the following embodiments, the features discussed with one embodiment may be extended to any of the other embodiments. The embodiments may include any of the features discussed in U.S. patent application no. 14/247,613, filed April 8, 2014, and published as U.S. patent application publication no. 2014/0303534, published on October 9, 2014.

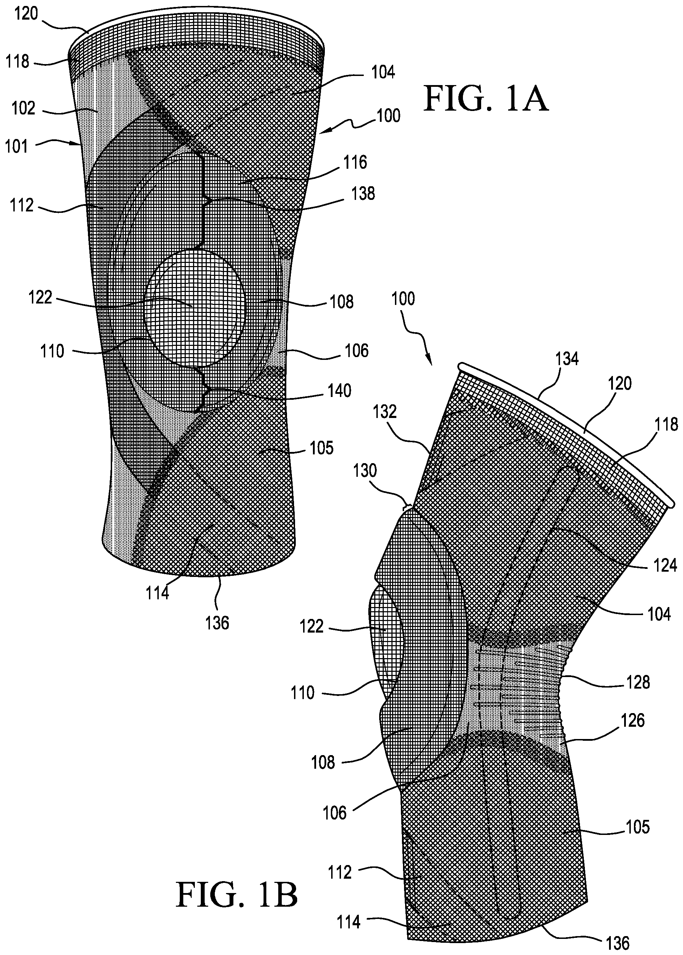

Referring to Figs. 1A and 1B, an orthopedic device **100** is arranged as a knee support, in this particular embodiment as a functionally knit orthopedic device. While the embodiment in Figs. 1A and 1B depicts a functionally knit orthopedic device, the teachings of the disclosure are equally applicable to other types of flexible orthopedic devices and orthopedic devices in general. The knee support **100** includes a main body panel arranged in this embodiment as a knit tubular sleeve **101** having first and second regions **102, 104, 105** with different elasticities. The first and second regions **102, 104, 105** are continuously knit to one another to have a continuous structure without interruptions. The first and second regions **102, 104, 105** are integrally knit to one another with the threads or yarns interwoven to one another rather than forming sections stitched to one another by additional threads or yarns. While integrally knit to one another, the first and second regions **102, 104, 105** may be considered discrete relative to one another because they are demarcated relative to one another by their properties, such as elasticity and/or color. Alternatively, the first and second regions **102, 104, 105** may gradually transition into one another due to sharing of certain properties of their knit, such as including shared elastic yarns. While in this embodiment the main body panel is arranged as a tubular sleeve **101,** other configurations and shapes may be suitable.

A tension band **112** is integrally formed and functionally knit with the first and second regions **102, 104, 105**. While the tension band 112 is functionally knit with the first and second regions 102, 104, 105 in this embodiment, tension band 112 may be adapted to be attached in any suitable way to other types of flexible orthopedic devices and orthopedic devices in general. The tension band **112** has a gradient region **114** dissipating into at least one of the first and second regions **102, 104, 105** to gradually taper into the first and second regions **102, 104, 105** toward first (proximal) and second (distal) ends **134, 136.** Between the first and second ends **134, 136,** the tension band **112** is preferably discrete in that it is clearly delineated from the first and second regions **102, 104, 105** without the dissipation. The tension band **112** may be arranged to extend about a patella region **108** of the knee support to improve support and tracking of the patella during physical activity.

The tension band **112** may lack or have minimal elasticity, such that it does not have or has only limited amounts of elastic yarns. Alternatively, the tension band **112** may be formed by yarns having more stiffness than yarns used to form the first and second regions **102, 104, 105.**

The tubular sleeve **101** may define a third region **106** having a different elasticity than the first and second regions **102, 104, 105.** The second region includes first (upper) and second (lower) portions **104, 105,** preferably formed adjacent the first and second ends **134, 136,** and operate as anchors to the dissipating portions **114** of the tension band **112.** They may have a greater rigidity or elasticity than the first region **102,** and may be on a medial or lateral side of the knee support **100.** The third region **106** is preferably between the first and second portions **104, 105** to enable better bending of the knee support **100** in view of the greater rigidity of the first and second portions **104, 105.**

The third region **106** includes a popliteal portion **126** and has a plurality of reinforcement bands **128** for modifying the flexibility of the popliteal portion **126.** The first and third regions **102, 106** may have generally the same elasticity at certain portions thereof. According to the embodiment of Figs. 1A and 1B, the third region **106** at the popliteal region **126** has a different elasticity than the first region **102** due to the reinforcement bands **128.**

The patella region **108** is separate from the first and second regions **102, 104, 105.** The patella region **108** forms an opening **110** and may have a panel **122** extending over the opening **110** to provide protection and proprioception for a user. The panel **122** may have elasticity greater than the first and second regions **102, 104, 105** so as not to inhibit bending of the patella. As an alternative, the panel **122** may have greater rigidity to restrict bending of the knee and offer enhanced support. The panel **122** may be continuously knit with the other features, or selectively removable from the tubular sleeve **101,** such as from an inner surface of the orthopedic device **100.**

The patella region **108** may have a bolster **116** formed around the opening **110** and protruding outwardly a distance **130** from a profile **132** of the tubular sleeve **101.** The bolster **116** may include padding embedded within the tubular sleeve **101.** The bolster **116** may taper toward the opening **110** and have a greater height away from the opening **110.** The bolster **116** may have a variable outer radius extending from the opening **110.** The bolster **116** may have a greater upper height **138** toward the first end **134** than a lower height **140** toward the second end **136** of the tubular sleeve **101.** The bolster **116** may include padding and reinforce and protect the knee of a user while functioning in combination with the tension band **112** to provide support to the user's knee during gait.

The tubular sleeve **101** may have a circumferential band **118** arranged about an open first end portion **134** of the tubular sleeve. The circumferential band **118** may have different elasticity than the first and second regions **102, 104, 105,** and preferably has significant elasticity with retaining properties functionally similar to a circumferential strap without the specific need for a strap to retain the knee support **100** on the leg of a user.

A binding **120** is provided about an open first end **134** of the tubular support **101** and terminates the circumferential band **118.** The binding **120** terminates the knee support **100** at a peripheral edge and protects the fabric of the knee support **100** from unraveling or damage. The binding **120** may be integrally knit with the circumferential band **118.**

A stay **124** is located between first and second ends **134, 136** of the tubular support **101** to provide support for articulation of a knee support **100.** The stay **124** may be embedded within the tubular support **101** or may be formed by stiffer knit or a combination of a support element such as a plastic strip and unique knit for forming the stay **124.**

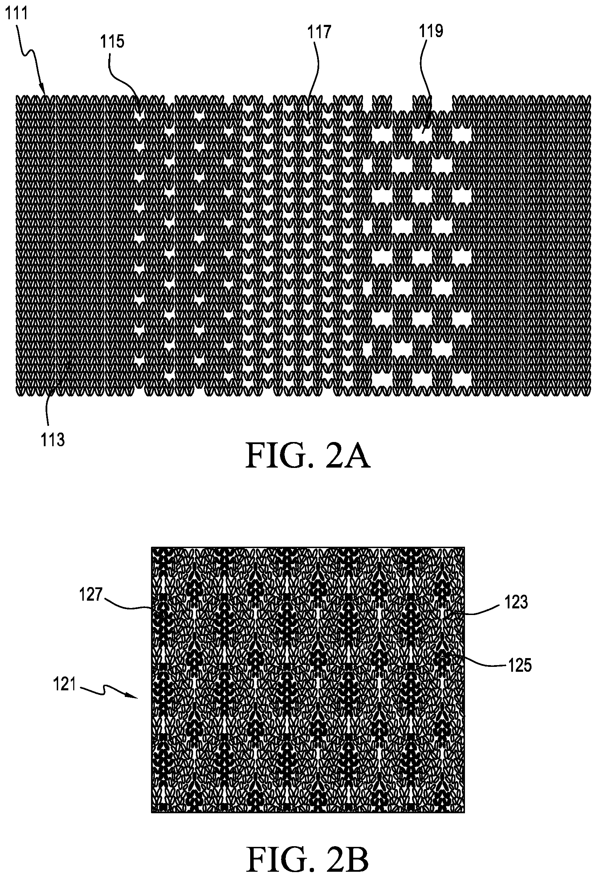

Fig. 2A shows an exemplary knit structure **111** formed from a knit material or knitting **113.** The knit structure **111** includes a plurality of apertures having different sizes **115, 117, 119** and formed from the knit material **113.** A first set of apertures **115** is smaller than second and third sets of apertures **117, 119** and has less elasticity than the second and third sets of apertures **117, 119.**

Fig. 2B shows another exemplary knit structure **121** having a plurality of different knit patterns **123, 125, 127.** A first knit pattern **123** surrounds a plurality of sections of second and third knit patterns **125, 127.** The first knit pattern **123** may have different properties than the second and third knit pattern **125, 127.** The first, second, and third knit patterns **123, 125, 127** may be distinguishable from one another on the basis of their color, elasticity, or a tightness of their knit.

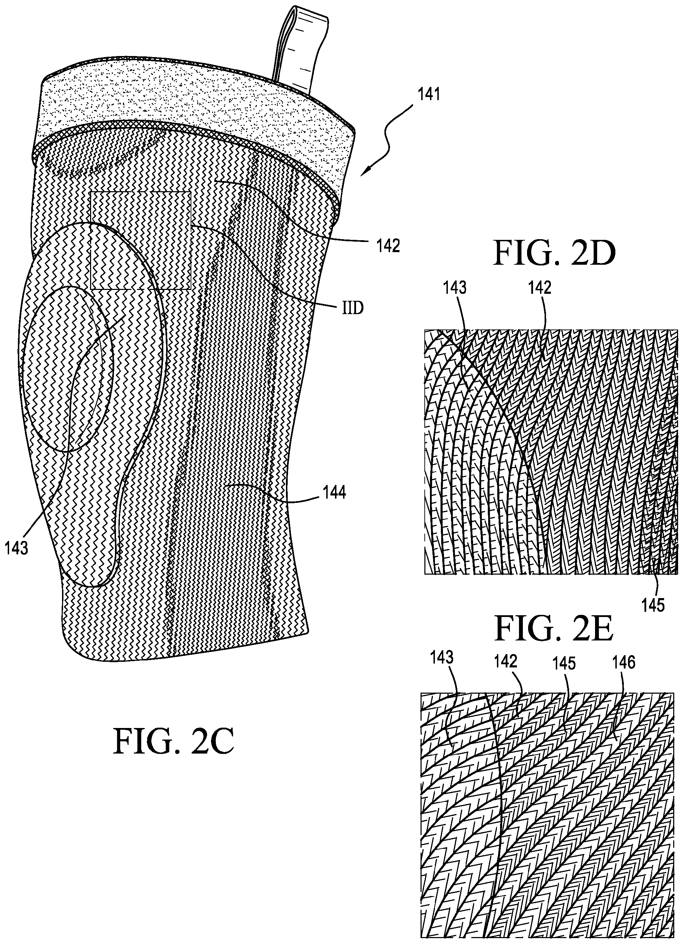

Fig. 2C exemplifies another knit structure **141** in a knee support **100.** In this embodiment, the knit structure **141** comprises different zones, such as first **142,** second **143,** and third **144** zones, that have different colors. Each zone **142, 143, 144** may have first and second layers of material **145, 146** (or outer and inner), which may be constructed from differently colored knitted structures, for example black and blue to obtain a visible contrast from each other.

For example, Fig. 2D shows the knee support **100** in extension whereby the first and second zones **142, 143** are not significantly tensioned over the knee. Fig. 2E shows the knee support **100** in flexion and tensioned or stretched over the knee whereby the contrast between the first and second zones **142, 143** is substantially more prominent due to a greater separation of discrete sections of the first layer **145,** such as columns or rows of the first layer **145** of material, and the revealing of the knitted structure of the second layer **146** between the first knitted structure **141,** in that one can easily observe a contrast between the first and second layers of material **145, 146,** whereas the contrast of such first and second layers **145, 146** of knitted material in Fig. 2D is minimal or non-existent.

The contrast of the layers offers a "shine-through" effect that provides a more aesthetic appearance of the knee support **100,** particularly as the knee support **100** undergoes tensioning and movement of the limb upon which it is worn. The shine-through effect offers a unique appearance to the knee support **100** that enables the manufacturer to distinguish its knee support **100** over other orthopedic devices. The contrast is not limited to colors of the knitted material, but can also relate to different finishes (stain, sheer, gloss) of the knitted material.

In this way, the first and second layers **145, 156** may further advantageously provide for different properties of moisture wicking, breathability, and heat transfer as required. For example, the first layer **145** may provide a tighter weave pattern to resist dirt and liquids from outside the orthopedic device to maintain cleanliness, while the second layer **146** may provide enhanced breathability for comfort. Alternatively, the first layer **145** may be configured to have less elasticity than the second layer **146,** such that when the knee support **100** is bent in flexion, the first layer **145** provides enhanced compressed against the joint. Numerous advantageous arrangements of the first and second layers **145, 146** can be similarly envisioned.

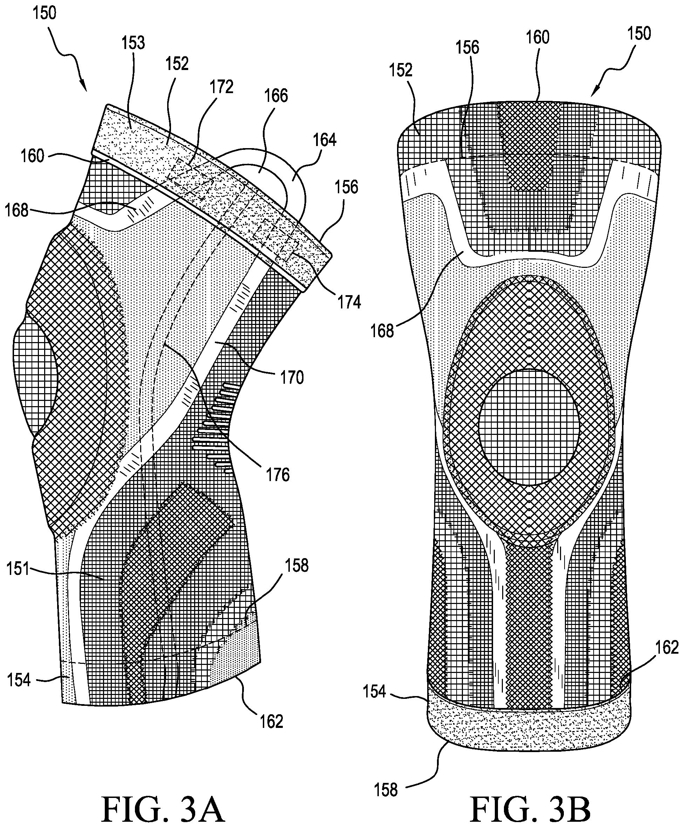

Figs. 3A and 3B illustrate a variation of the orthopedic device of Figs. 1A and 1B. In the orthopedic device **150,** an upper cuff **152** is secured to a tubular body **151.** The upper cuff **152** is arranged to provide migration control, which may be accomplished in a variety of ways, but is nonetheless to be disengaged during donning, doffing, and fitting of the orthopedic device **150** such that the orthopedic device **150** may be easily donned or doffed without sacrificing the advantages of the migration control.

In the illustrated example, the upper cuff **152** defines a frictional surface from a frictional material **153** that is arranged to engage the skin of the wearer and maintain the tubular sleeve **151** on the leg. An example of the frictional material **153** is silicone that is applied to the inner surface of the cuff **152** (when engaging the skin). Alternatively, the upper cuff **152** may be arranged or suitably knit in a manner that provides radial compressive forces about the leg.

The upper cuff **152** is adapted to flip over an upper end portion of the tubular sleeve **151** during donning. For example, upper cuff **152** defines a first edge **160** which forms an upper edge to the orthopedic device **150** when the upper cuff **152** is in an engaged configuration for normal use (as in Fig. 3B), and the first edge **160** is arranged to flip over the tubular sleeve **151** when the upper cuff **152** is in a donning/doffing or disengaged configuration (as in Fig. 3A). A folding portion **156** forms a joint about which the upper cuff **152** folds over the tubular sleeve **151,** and generally demarcates where the frictional material **153** terminates. The upper cuff **152** is arranged such that when the upper cuff **152** is folded over the tubular sleeve **151,** the upper cuff **152** is adjacently against the tubular sleeve **151,** wherein an outer surface of the upper cuff **152** snugly abuts a portion of an outer surface of the tubular sleeve **151** due to its radially compressive construction.

The orthopedic device **150** may include a lower cuff **154** arranged similarly to the upper cuff **152,** for migration control of the orthopedic device **150** when in an engaged configuration. The lower cuff **154** may include a frictional material, and is arranged to flip over the tubular sleeve **151** about folding portion **158.** When in an engaged configuration, the first edge **162** of the lower cuff **154** forms a lower edge of the orthopedic device **150.** When in a disengaged configuration, the folding portion **158** forms a lower edge of the orthopedic device **150.**

In order to facilitate donning of the orthopedic device **150,** handles **164** may be provided so as to extend from the tubular sleeve **151** to assist in pulling the orthopedic device **150** about a leg. As the knitted structure of the tubular sleeve **151** is intended to snugly secure about the leg, and in view of the need to tension the orthopedic device **150** about differing circumferences of the leg, the handles **164** enable the user to position the orthopedic device **150** in a suitable location to maximize the benefit of the orthopedic device **150.** The handles **164** may include an opening **166** for insertion of fingers to aid the user in gripping the handles **164,** which is particularly advantageous when the user is infirm or there are significant circumference differences of the user's leg, as handles **164** offer a user better purchase than trying to grip the tubular sleeve

**151.**

The handles **164** also advantageously prevent a user from pulling directly on the tubular sleeve **151,** thus preventing damage thereto. Pulling on handles **164** rather than tubular sleeve **151** is also more intuitive and simple for users, as doing so is less likely to result in undesired rotation of the orthopedic device **150** during donning. By locating the handles **164** on an inner surface of the tubular sleeve **151,** damage is averted as the handles **164** are less likely to accidentally catch on objects as a user moves about, and the upper cuff **152** is better able to fold over the tubular sleeve **151** for donning and doffing without interfering with the use of the handles **164.**

The tubular sleeve **151** may define band segments **168, 170** that are reinforced or have an elasticity or omit elasticity in line with the contours of the handles **164.** For example, the band segments **168, 170** are inelastic or substantially inelastic so the tubular sleeve **151** can be suitably, sufficiently, and generally uniformly tensioned about the leg, as it is readily understood from the other embodiments described herein that there are elastic and flexible regions belonging to the orthopedic device **150.**

The handles **164** may be extensions of the knitted band segments **168, 170** and may flexibly extend from the tubular sleeve **151,** or alternatively may be secured to the tubular sleeve **151** such as along the upper folding portion **160.** In a variation, as depicted in Fig. 3A, the handles **164** may be slidably mounted to the tubular sleeve **151** by internal loops **172, 174** on the tubular sleeve **151.** When not in use, the handles **164** may retract relative to the tubular sleeve **151,** and when desired for use, the handles **164** can be extended relative to the tubular sleeve **151.** Particularly, if an upper cuff **152** is not provided with the orthopedic device **150,** the handles **164** can still be used for donning the orthopedic device **150.**

The orthopedic device **150** includes a stay **176** generally formed by reinforced knitting on the tubular sleeve **151.** The stay **176** may be knitted over the tubular sleeve **151** by different, stiffer and resilient yarns or a combination thereof, or may be knitted together with the remainder of the tubular sleeve **151.**

The orthopedic device **150** omits features in a region directly adjacent to upper cuff **152** such that upper cuff **152** can be folded over to be adjacently against tubular sleeve **151** without interference from other features.

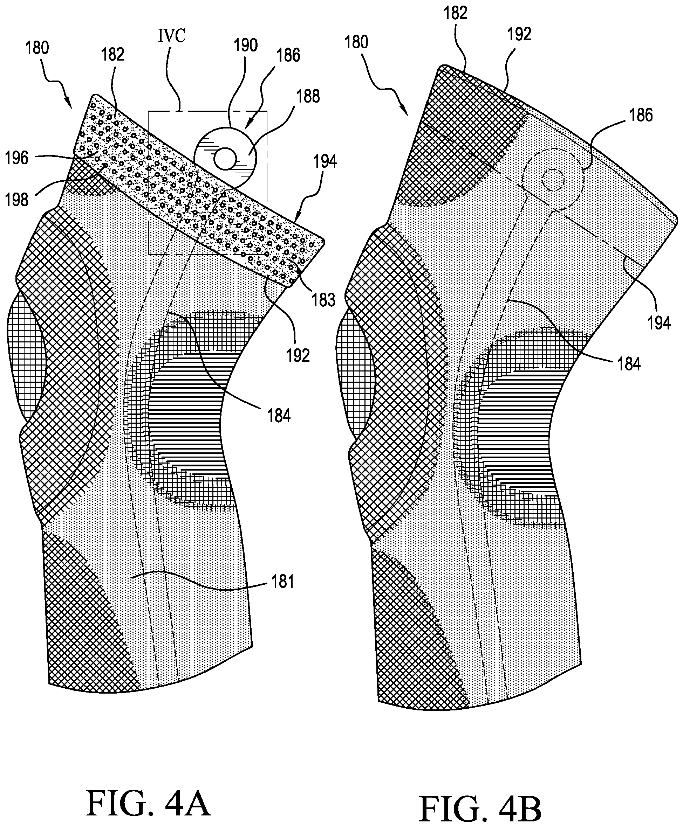

Figs. 4A and 4B represent an embodiment of an orthopedic device **180** according to the invention having an upper cuff **182** in combination with a resilient stay **184** that is provided in combination with a main body panel arranged in this embodiment as a tubular sleeve **181.** In this embodiment, the upper cuff **182** includes a frictional material **183** located on a surface thereof and arranged to engage the skin of the user similarly to the embodiment of Figs. 3A and 3B. The stay **184** is formed by plastic or metal and has resilient properties adapted to resisting flexion of the orthopedic device **180,** and extends between upper and lower ends of the orthopedic device **180.** The stay **184** incorporates at its upper end a handle **186** that is integrated therewith. The handle **186** includes a head portion **188** configured for facilitating grasping with padding **190** located along a surface of the handle **186** adjacent the user's skin.

By incorporating the handle **186** with the stay **184,** preferably on medial and lateral sides of the orthopedic device **180,** rotation of the orthopedic device **180** about the user's leg can be prevented during donning. Indeed, once the upper cuff **182** is flipped about an upper folding portion **194** into an engaged configuration with an upper cuff edge **192** forming the upper edge of the orthopedic device **180,** the upper cuff **182** prevents migration and rotation of the orthopedic device **180** on the leg. Contrariwise, when the upper cuff **182** is in a disengaged configuration, the upper cuff **182** is folded over tubular sleeve **181** so as to be adjacently against the tubular sleeve **181,** due to the upper cuff **182** having radially compressive properties. In this configuration, an outer surface of the upper cuff **182** snugly surrounds a portion of the outer surface of the tubular sleeve **181,** which is possible due to other features of the orthopedic device **180** being spaced downwardly from the upper cuff **182.**

The frictional material **183** may be provided in different forms. In the example of Fig. 4A, the frictional material **183** defines a band **196** that generally circumferentially extends about the inner surface of the upper cuff **182.** The band **196** may include a texture **198** that may form a plurality of openings, raised surfaces, random texture, or other textures that improve comfort for the user. In the embodiment of Fig. 4A, a preferred frictional material **183** is silicone, in part due to its skin adhering properties and it enabling gentle roll-off when doffing the orthopedic brace **180** and therefore folding the upper cuff **182** over the tubular sleeve **181.** Other materials are envisioned providing similar properties.

In an alternative embodiment, the cuffs in the aforementioned embodiments may be formed by knitted silicone. As silicone has skin tackiness properties enabling gentle roll-off and roll-on, while having proven migration control characteristics, any of the embodiments may include silicone knit regions, whether by intarsia, terry stitch, waffle, circumferential areas, or other suitable applications. An example of silicone knitting is discussed in U.S. patent application no. 15/219,772, filed on July 26, 2016.

Uses of the knitted silicone include suspension, as in the aforementioned embodiments, massaging effects, and capture of bony and muscle anatomies. For suspension the knitted silicone may be at the top and/or bottom of a tubular brace, as in the upper and lower cuffs. The knitted silicone may be located along the length of the tubular brace, in which case it is best to have it in the same plane as the bending of the joint occurs, where shortening/lengthening does not occur. For example, in the knee, this would be along the medial and lateral side.

In another use of knitted silicone, the knitted silicone areas may provide a massaging effect, such as around the Achilles tendon, in the case of Achilles tendonitis. Silicone knit areas can be used for massaging, as the silicone knit areas are soft and do not exert any painful pressure, while evenly distributing pressure. The massaging effect may be provided by intarsia or terry knit in desired areas of the orthopedic device, while increasing in grade of an area to increase blood flow.

The knitted silicone can be arranged for capturing key bony anatomies through the surface of the skin. The silicone area can capture, for example, the patella of the knee by relying on the compression and frictional features and localizing the silicone area about the patella.

The knitted silicone can be used to capture key anatomies, and can be knitted into the orthopedic device to take place of Kinesio-taping or as elastic therapeutic tape built into the knitted structure of the orthopedic device as a whole. For example, in the embodiments described herein, one of the zones or regions of different elasticity may include a zone or region that is formed by knitted silicone so as to stretch beyond the original length of the zone to create a pulling force on the skin by the frictional properties and recoiling of the knitted silicone zone when the orthopedic device is worn. Thus, knitted silicone zone can be pretensioned or otherwise modified to create traction or pulling forces on the skin in combination with its adhesive properties.

As understood in Kinesio-taping applications, the knitted silicone zones may have general shapes for different taping type uses. For example, an "1" shape may be used for small or linear places, a "Y" shape may be employed for larger muscles, and an "X" shape may be used for long and large muscles.

Knitting in silicone presents two major challenges: first, the silicone is very sticky, and second, the silicone is highly elastic. Taken together, these challenges make it difficult to knit with silicone. The reason is that when knitting, the stickiness of the silicone causes it to stretch greatly in an uncontrolled manner. Since yarn stretch is crucial to form when knitting, the finished product will be greatly variable, and the manufacturing unreliable. To prevent this, one can either limit the elasticity of the silicone or make it less sticky.

One way to limit the elasticity of the silicone is to extrude it around a nylon core. The nylon core fabric can be spun in such a way as to have elasticity ranging from 0% (completely inelastic) to 100% (able to stretch to double its length at rest) or beyond. This method permanently limits the elasticity of the silicone yarn.

Another way to limit the stickiness of the silicone is to spin a non-sticky yarn around the silicone core. The silicone yarn can then be freely knitted. The external yarn is then removed post-knitting, for example by dissolving it in hot water or through some chemical process. In this method, the silicone retains its stickiness and elasticity.

Yet another way of limiting the stickiness of the silicone is to coat it in an oil or non-sticky powder prior to knitting. This will reduce the stickiness of the silicone. However, like the previous method, the oil/powder coating will need to be removed post-knitting. Possibly, the method of removal may be as simple as washing the product in a washing machine.

As discussed above, parts of the orthopedic device can be knitted with silicone. This can be done in intarsia, where certain areas will then include silicone for functional benefits. This can also be done not in intarsia but in circumferential zones such as at the top and bottom edges.

The silicone knit can be done with multiple types of knit stitches. One stich type may result in the silicone creating a relatively uniform area of contact. It may be beneficial to have only limited contact with silicone, so another stitch may only expose part of the silicone yarn, and create "dots" of silicone in the garment. It may also be beneficial to have as much contact with silicone as possible, so to create larger surface area, the stitch may be a so-called "terry" stitch.

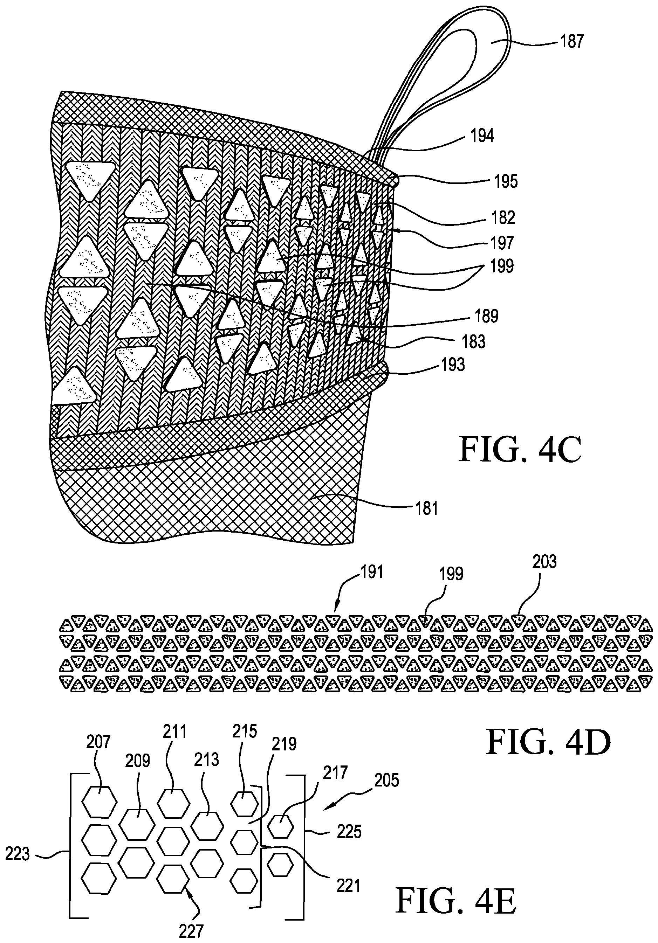

Fig. 4C exemplifies a variation of the upper cuff **182** in the folded or disengaged configuration, as in Fig. 4A. In this embodiment, the handle **186** is a flexible loop **187** stitched generally along the upper folding portion **194.** A lower band **195** may be stitched or otherwise formed proximate the upper folding portion **194** to demarcate or delimit the folding of the upper cuff **182.** Lower band **195** and upper folding portion **194** advantageously function to signal to a user where the upper cuff **182** is to be folded, so that the upper cuff **182** and the attached frictional material **183** are (preferably) adequately and completely disengaged from the user's leg when folded over. The loop **187** may be anchored along or proximate the lower band **195.**

The upper cuff **182** has an upper band **193** that may have greater elasticity and reinforcement so as to apply greater circumferential pressure along a limb than the remainder of the upper cuff **182** or tubular sleeve **181.** For example, the upper band **193** may be provided with more elastic elements when knitted, may have a tighter knitted structure, or may have a thicker knitted structure. Additionally, the upper band **193** advantageously allows an outer surface of the upper cuff **182** to be adjacently against an outer surface of the tubular sleeve **181** when in the disengaged configuration, in that the upper band **193** may assist the outer surface of the upper cuff **182** to snugly abut or surround a portion of the outer surface of the tubular sleeve **181** when folded over, in part due to the radial compression of the upper cuff **182** and in particular the upper band **193.** The lower band **195** may likewise be modified to have a different knitted structure to facilitate folding and demarcating the extent of the folding of the upper cuff **182** over the tubular sleeve **181.**

To further facilitate the folding of the upper cuff **182** into the disengaged configuration, or otherwise the extent to which the upper cuff **182** is folded over the main body of the tubular sleeve **181,** at least an interior surface **197** of the upper cuff **182** may have a different color or knitted pattern than the tubular **181;** for example, the tubular sleeve **181** may be black whereas the interior surface **197** is light blue.

While an upper cuff **182** is described, a lower cuff may be similarly structured in order to facilitate donning and doffing; that is, the lower cuff may likewise comprise bands specially configured to facilitate folding and demarcating the extent of the folding of the lower cuff over the tubular sleeve **181** in order to engage and disengage frictional elements. By configuring either or both of an upper cuff and a lower cuff to be engaged or disengaged at will by a user to facilitate donning/doffing of the orthopedic device, a key setback in orthopedic devices is overcome: the orthopedic device is able to be easily donned and doffed due to the disengaged configuration (wherein the frictional elements along the upper and lower cuffs do not abut the user's anatomy), but in the engaged configuration the frictional elements overcome the tendency of such devices to migrate along a user's leg, which tendency hinders the effectiveness of the device.

The interior surface **197** includes a frictional material **191** arranged in a pattern to balance friction between the upper cuff **182** and the intended limb of the wearer of the knee support with comfort and breathability for a user. For example, the frictional material **191** has a pattern of a circumferential array of discretely deposited sections **199** that are shaped to coincide of the knitted structure of the upper cuff knitted pattern **189.** In this example, the frictional material pattern is similar to columns of opposing triangles that generally follow columnar aspects of the knitted pattern **189.** In this example, the sections **199** are arranged so as not to impede breathability. The columns of the pattern of deposited sections **199** extend circumferentially about the interior surface **197** of the upper cuff **182,** leaving sufficient space between each column to ensure breathability and comfort by balancing the exposure of the knitted structure and frictional material **191** against the skin of the user.

While the frictional material **191** is shown as having a pattern that generally corresponds to the knitted structure of the upper cuff **182,** at least along the interior surface **197** thereof, the frictional material **191** may be provided in a pattern that does not correspond to the knitted structure.

Fig. 4D exemplifies a band **203** that can serve as a frictional material **191** that is deposited along the interior surface **197** of the upper cuff **182.** The pattern of the frictional material **191** may or may not correspond to the upper cuff knitted pattern **189.** The band **203** may be formed separately from the knitted structure, and later applied to the interior surface **197** of the upper cuff **182,** whether or not corresponding to the pattern **189** of the knitted structure. The band **203** has sufficient spacing among discrete regions to ensure sufficient breathability for the user in that the pattern is arranged so as to generally universally correspond to knitted structures while not substantially impeding airflow.

Fig. 4E is another example of a pattern **205** of frictional material. In this example, the pattern **205** defines a plurality of columns **207, 209, 211, 213, 215, 217** of discrete frictional elements **227.** In this embodiment, the pattern **205** has boundaries **223, 225** within which the pattern **205** is located, which lends itself to being applied only to discrete segments of a circumference of the upper cuff **182.** Alternatively, as in a cuff, the pattern may be provided to circumferentially extend about a cuff. In a preferred embodiment, the pattern 205 of frictional elements 227 comprises and/or is constructed of a knitted silicone or other tacky type thread.

The frictional elements **227** may be variably sized relative to one another in each column, and may comprise different spacings **219, 221** relative to one another, particularly if the pattern **205** is located on areas of an orthopedic device **200,** not just an upper or lower cuff, that require variable friction. In areas of greater need for frictional control, the frictional elements **227** may be larger and/or located closer to one another, and in areas with less need for frictional control the frictional elements may be smaller and spaced apart greater. In any situation, however, the variability of the pattern **205** may be adapted to variable frictional and migration control needs in an orthopedic device.

Even though frictional material may be contained in a cuff, of the type described above, it is often desirable for additional circumferential frictional control to prevent migration of the knee support on the leg of the user. Certain areas of the leg will lend themselves to including more migration control than others; for example, the anterior and posterior sides of the leg may require more frictional control, particularly in flexion and extension, than the medial and lateral sides of the leg.

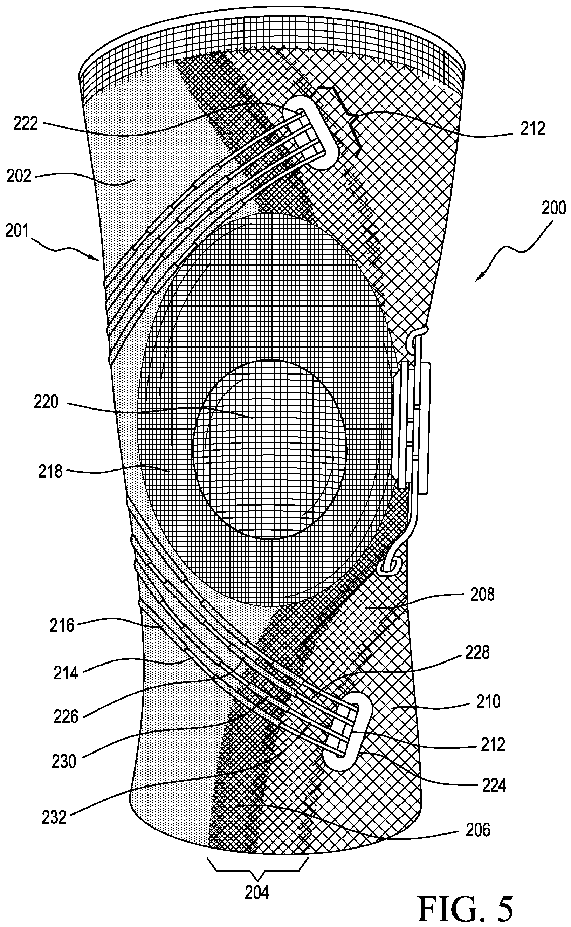

In another embodiment depicted in Fig. 5, an orthopedic device **200** in a knee support includes a main body panel arranged in this embodiment as a knit tubular sleeve **201** having first and second regions **202, 204** with different elasticities. The first and second regions **202, 204** are continuously knit to one another. The second region **204** includes at least first and second gradient segments **206, 208** arranged for modifying elasticity of the tubular sleeve **201** from the first region **202.** The knee support **200** may include a third region **210** having different elasticity from the first and second regions **202, 204,** and may resemble the first and second portions **104, 105** of the embodiment of Figs. 1A and 1B.

The knee support **200** may include a patella region **218** defining an opening **220** in that each of the first and the second regions **202, 204** extend discretely about at least a portion of the patella region **218.** The patella region **218** may resemble the patella region **108** of the knee support **100** of Figs. 1A and 1B.

The knee support **200** includes a cable system **212** having at least one cable **214** slidably movable relative to the tubular sleeve **201.** At least one guide **216** may be integrally formed from the tubular sleeve **201** and is arranged for receiving the at least one cable **214** such that the at least one cable **214** slides within the at least one guide

**216.**

In the embodiment, the cable system **212** is on an opposite side of the patella region as the first region **202.** The second region **204,** whether with or without the gradient **206, 208,** and the cable system **212** may interact with one another and may be tailored or adjusted to offer the requisite support for a user's patella.

The cable system **212** may include an adjustment system (not shown) that enables tensioning of the cable system **212.** Examples of adjustment systems may be found in U.S. patent application publication no. 2014/0303534, U.S patent application publication no. 2013/0211304, published on August 15, 2013, U.S. 2015/0032041, published on January 29, 2015, and U.S. patent application publication no. 2015/0121657, published on May 7, 2015.

A slot **222** in the tubular support **201** is arranged for receiving a segment of the at least one cable **214.** The at least one cable **214** is configured to have a segment received within a thickness of the tubular sleeve **201,** and the adjustment system may take up or dispense a length of the at least one cable **214.** The slot **222** may be reinforced with a reinforcement element **224** secured to the tubular sleeve **201** about the slot **222.**

The cable system **212** preferably includes a plurality of cables **214** slidable relative to the tubular sleeve **201.** A plurality of guides **216** are secured to a surface of the tubular support **201** and each is arranged for receiving an individual one of the cables **214.** First and second guides **226, 230** may each be along a different segment of first and second cables **228, 232,** and the first and second guides **226, 230** may have different lengths. The cable system **212** is preferably configured in a predetermined configuration over a surface of the tubular support **201** such that the at least one cable **214** is constrained in location over the tubular support **201** by the at least one guide **216.**

The cable system **212** solves the problem of straps being difficult to use or adjust, and being cumbersome due to the straps straying from their intended location, tangling, or being damaged by external forces. By providing the cable system **212** with at least one cable **214** and at least one guide **216** defined by and/or integral with the tubular sleeve **201,** the problems of straps are avoided but the beneficial purposes thereof (i.e. compression in specific locations, unloading of joints, securely mounting the device on the limb) may be retained.

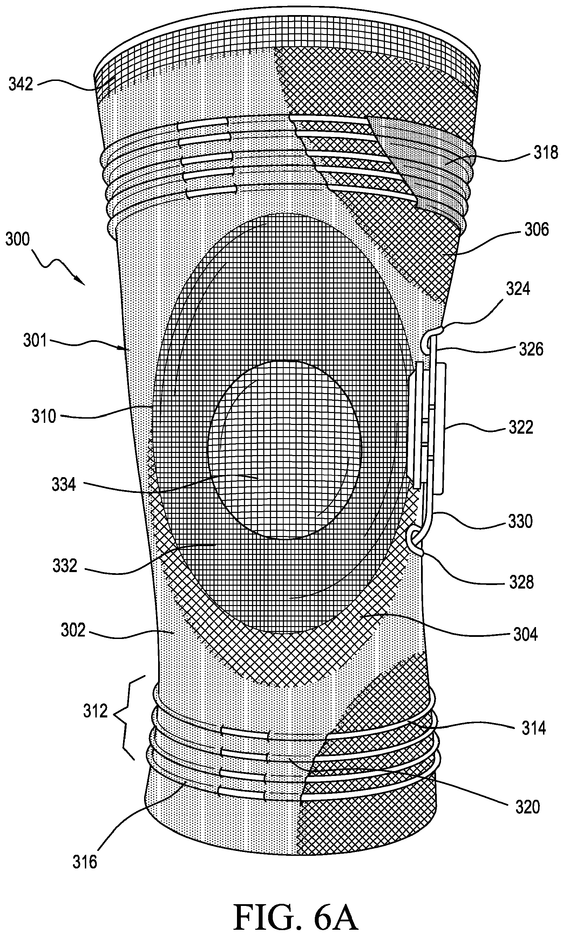

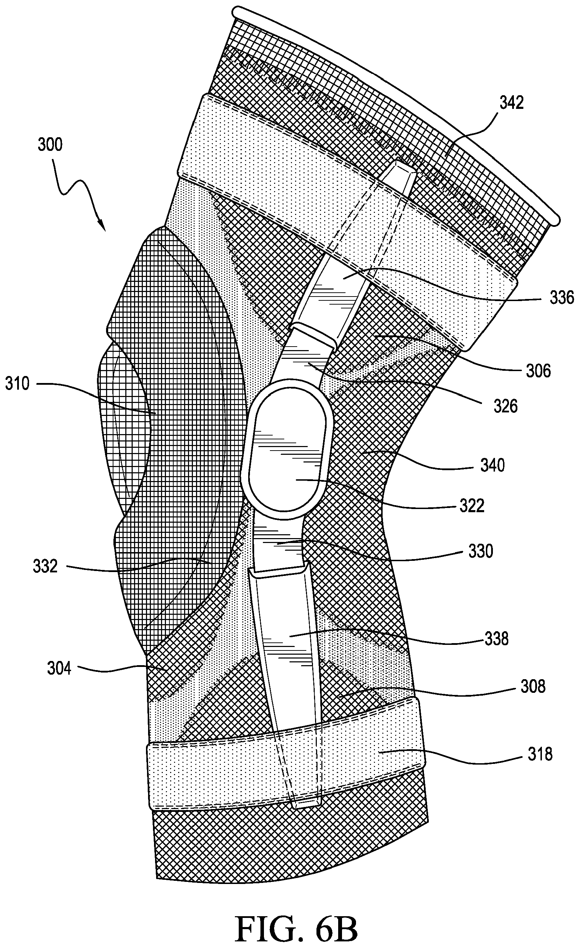

Figs. 6A and 6B illustrate another orthopedic device embodiment in a knee support **300.** As with foregoing embodiments, the knee support **300** includes a main body panel arranged in this embodiment as a knit tubular sleeve **301** having first and second regions **302, 304** with different elasticities. The knee support **300** has a patella region **310** defining an opening **334,** with each of the first and the second regions **302, 304** extending discretely about at least a portion of the patella region **310.** A bolster **332** is formed around the opening **310,** and the second region **304** extends from the bolster **332** toward an end portion of the tubular sleeve **301.**

The knee support **300** further has a hinge assembly **322** secured to the tubular support **301.** To accommodate the hinge assembly **322,** the tubular support **301** has first and second strut inserts **336, 338** secured to the tubular sleeve **301.** The hinge assembly **322** has first and second struts **326, 330** received by the first and second strut inserts **336, 338.** The strut inserts **336, 338** may be formed from a plastic material and laminated or stitched to the tubular support **301,** being disposed along an external surface of the tubular support **301.** The struts **326, 330** may be removable from the inserts **336, 338,** and the inserts **336, 338** offer selective placement of a desired hinge assembly **322.**

In a variation of the hinge assembly **322,** the first and second strut inserts **336, 338** are within a thickness of the tubular sleeve **301.** First and second openings **324, 328** are arranged for receiving first and second struts **326, 330** into the tubular sleeve

**301.**

The tubular sleeve **301** may have a third region including first and second portions **306, 308** corresponding to the first and second struts **326, 330.** The first and second portions **306, 308** may have elasticity less than the first region **302,** and may resemble similar structures in foregoing embodiments. As with the foregoing embodiments, the first and second portions **306, 308** are discretely separate from one another. A popliteal region **340** is preferably between the first and second portions **306, 308** and has elasticity greater than the first and second portions **306, 308.**

The orthopedic device **300** has a cable system **312** including at least one cable **314** slidably movable relative to the tubular sleeve **301,** as in foregoing embodiments. The cable system **312** has at least one guide **316** connected to the tubular sleeve **301** and is arranged for receiving the at least one cable **314** such that the at least one cable **314** slides within the at least one guide **316** and is routed over the tubular sleeve **301.** The at least one guide **316** includes a plurality of guide segments **320** spaced apart from one another and exposing the at least one cable **314** therebetween.

A guide cover **318** covers an entirety of a segment of the at least one guide **316.** The cover **318** may be constructed as an adjustment device or anchor for the at least one cable **314,** and be adjustable in location over a surface of the tubular support **301.** The cover **318** may have a fastener, such as hook and loop, engageable with a corresponding fastener on the tubular support **301.**

As shown in Figs. 6A and 6B, the cable system **312** is arranged circumferentially about the tubular support **301,** and takes the place of a strap found in a conventional brace. The cable system **312** may cooperate with a circumferential band **342** to retain the knee support **300** on a leg of user, with the cable system **312** providing additional support to the circumferential band **342.** The cable system **312** may extend over the hinge assembly **322** to maintain the circumferential support without impeding use of the hinge assembly **322.** Alternatively, the cable system **312** may be routed underneath the hinge assembly **322** to exert circumferential tension over the leg of the user.

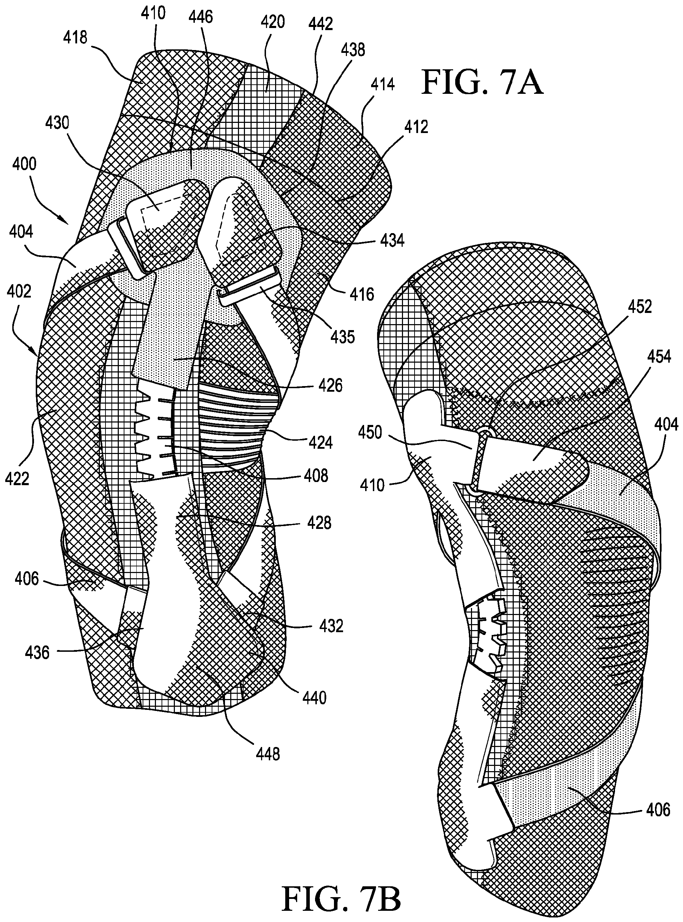

Figs. 7A and 7B illustrate another embodiment of an orthopedic device in this embodiment in the form of a functionally knit knee support **400** aimed for treating osteoarthritis of the knee. While the orthopedic device in this embodiment is a functionally knit orthopedic device, the teachings of the disclosure are equally applicable to other types of flexible orthopedic devices and to orthopedic devices in general. The knee support defines a main body panel arranged in this embodiment as a tubular sleeve **402,** and has first and second straps **404, 406** arranged to spiral about an outer surface of the tubular sleeve **402,** and intersect at a predetermined location. The operation and purpose of the straps **404, 406** for treatment of osteoarthritis of a knee are described in U.S. patent 7,198,610, granted April 3, 2007. The location of the intersection of first and second straps **404, 406** advantageously overcomes the problems of existing straps which intersect over a hinge directly on a medial or lateral side of the brace, which location is not effective for treating osteoarthritis.

The knee support **400** includes a hinge support **410** located along at least one side of the knee support **400,** and extends into upper and lower regions of the knee support relative to a central or knee portion. The hinge support **410** is arranged to support or position a hinge **408,** and provides anchor areas **446, 448** for the first and second strap **404, 406.** The hinge support **410** defines sleeves **426, 428** for receiving portions of the hinge **408** to secure the hinge **408** relative to the tubular sleeve **402,** and enable removal of the hinge **408.** For example, a different type of hinge, such as a hinge having different stiffness or articulation (four-bar, polycentric, biasing bar), may easily replace an existing hinge.

The hinge support **410** may serve as anchor points for the first and second straps **404, 406.** According to the illustrated embodiment, the hinge support **410** is located along either the medial or lateral sides of the knee support **400,** and generally along the anterior-posterior plane. Indeed, the hinge support **410** extends between upper and lower regions of the tubular sleeve **402** along one of the medial and lateral sides of the knee support **400.** The hinge support **410** may be articulated at the anterior and posterior knee portions **422, 424** of the tubular sleeve **402** to accommodate a natural contour of a leg.

The hinge support **410** may be preferably more rigid than the tubular sleeve **402,** although it may be pliant to permit easy contouring of the knee support **400** to the leg of the user. Nonetheless, the hinge support **410** preferably provides greater stability for securing elements, such as straps and a hinge, than the knitted tubular support **402.**

In an example, the hinge support **410** is formed from a material having greater rigidity than the knitted structure of the tubular sleeve **402**. By way of example, lower or second ends **432, 436** of the first and second straps **404, 406,** are permanently or removably secured to a lower or second portion **448** the hinge support **410.** By "permanently" secured, it is intended to mean that it is not readily removable by a user, and from normal usage of the knee support **400,** a user cannot remove the lower end from the tubular sleeve **402** without significant difficulty. By "removably" secured, it is intended to mean during normal use a user can detach the lower end **432, 436** from the hinge support **410.**

In the configuration of being permanently secured, the lower ends **432, 436** may be pivotally secured to the hinge support **410,** and extend upwardly in a spiraling manner to an upper or first portion **446** on the hinge support **410.** Upper or first ends **430, 434** of the first and second straps **404, 406** are removably secured to the upper or first portion **446** of the hinge support **410.** Preferably, the upper or first portion **446** of the hinge support **410** comprises hook receivable material and the upper or first ends **430, 434** having corresponding hook material engageable with the upper or first portion **446.** To guide the first and second strap **404, 406,** a guide **435,** such as a loop, is proximate or at the upper or first portion **446.** The upper or first portion **446** is sufficiently sized to accommodate both upper or first ends **430, 434** without overlap.

The upper and lower portions **446, 448** may include first and second stays **438, 440** to provide reinforcement to the straps **404, 406,** and possible tension they may exert on the tubular sleeve **402.** The stays **438, 440** may be semi-rigid to provide additional support beyond the hinge support **410** and material of the tubular sleeve **402.** The stays **438, 440** may be permanently secured or removably secured to or within the hinge support **410.** The hinge **408** may overlap the stays **438, 440** whereby the stays **438, 440** may cover a greater portion of the tubular sleeve **402** than hinge arms of the hinge **408,** and may serve to distribute pressure exerted by the hinge arms.

The tubular sleeve **402** defines different regions of elasticity, wherein the tubular sleeve **402** includes a main panel **416** that generally possesses a baseline elasticity to comfortably and functionally support a leg. The tubular sleeve **402** includes an upper cuff **414** that may function and be structurally arranged similarly to aforementioned embodiments. The upper cuff **414** preferably has a lower band **412** that is located above the hinge support **410** so the hinge support **410** and stays **438, 440** it may contain do not interfere with folding the upper cuff **414** between the engaged and disengaged configurations, with a proximal edge **442** extending over the hinge support

**410.**

The tubular sleeve **402** may include a first region of elasticity **418,** as in preceding embodiments, on the anterior side of the knee support **400,** and may be more rigid or having a contour to embrace or add further support to the knee over the main panel **416.** The tubular sleeve **402** may also include a second region of elasticity **420** along or in line with the hinge support **410.** The second region of elasticity **420** may be more rigid than the main panel **418,** and operates with the hinge support **410** to provide greater rigidity along a direction in which the hinge **408** extends.

Referring to the embodiment of Fig. 7B, a variation of the knee support **400** of Fig. 7A includes angled extensions **450** extending from the hinge support **410** to accommodate the spiraling and angling of the first and second straps **404, 406.** A D-ring or other suitable attachment **452** extends from the extensions **450** and permits the first and second straps **404, 406** to secure over itself at first ends **454** to set the length of the straps **404, 406.** Although not depicted, the second ends of the straps **404, 406** may be similarly modified to secure onto themselves.

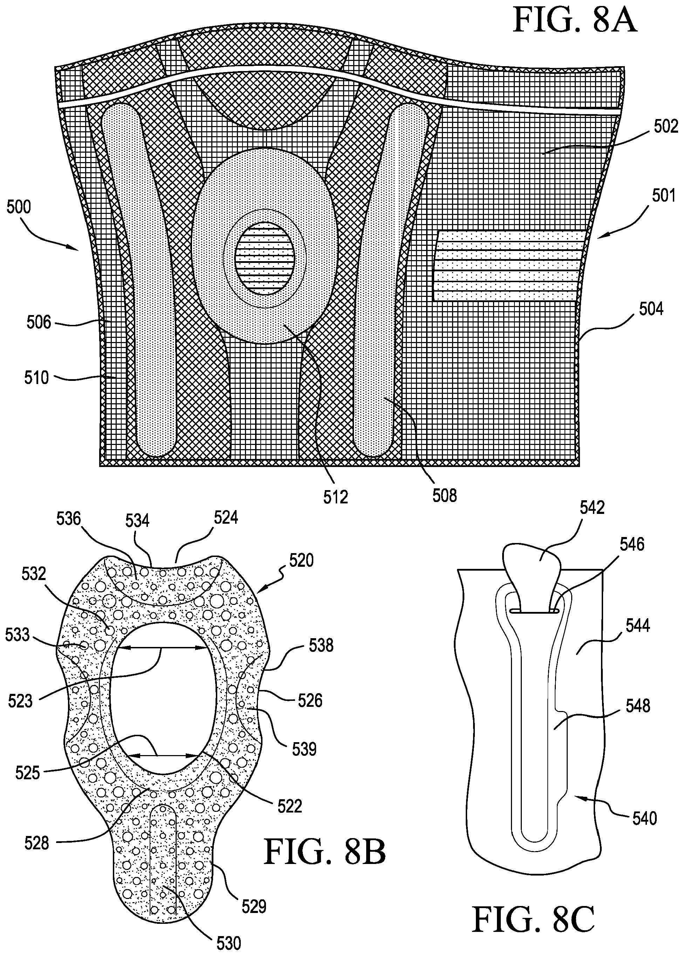

Fig. 8A depicts an exemplary embodiment of an interior surface **502** of a main panel **501** of a knee support **500.** In this embodiment, the main panel **501** may be securable along longitudinal side portions **504, 506** to form a tubular structure. The interior surface **502** may support various stays, bolsters and pads, including stays **508, 510,** and a patella pad **512.** These stays, bolsters and pads may be removably secured or permanently secured to the main panel **501.**

Fig. 8B depicts an exemplary patella pad **520** that may be removably or permanently secured to a knee support **500.** The patella pad **520,** as depicted showing an outer surface with the inner surface being generally planar in a flat configuration and adapted to be secured to the tubular sleeve of a knee support **500,** has peripheral relief portions **524, 526** that are provided for reducing pressure and improving flexure according to flexion and extension of a knee.

The top relief portion **524** has an arcuate cut-out **534** along the periphery and a corresponding concave relief **536.** This configuration provides pressure relief on top of the patella and negates pressure on the knee from above the patella. The lateral relief portions **526** likewise have an arcuate cut-out **538** and a corresponding concave relief **539,** which provides for better flexure of the patella pad **520,** and anatomically contours to the side of the knee during flexion of the knee.

The patella pad **520** defines an opening **522,** that may preferably have an oval and non-uniform shape with a greater width at an upper portion **523** and a narrower width at a lower portion **525,** to better receive and anatomically contour to the shape of a patella. Below the lower portion **525,** there is a segment formed as a patella tendon support zone **528** that leads to a lower extension **529** having an elongate recess **530** or concave relief on the patella ligament. The elongate recess **530** is recessed relative to the patella tendon support zone **528,** which is formed in part by a generally circumferential width of the patella pad **520** about the opening **522.** This structure advantageously allows the patella pad **520** to provide adequate support to a user's tibial tuberosity but mitigates undesired pressure points by means of the elongate recess **530.**

The generally circumferential width gradually tapers to the opening **522** or an inner periphery of the patella pad **520,** and yet further tapers to the outer periphery of the patella pad **520,** along which the relief portions **524, 526, 536** are located. The relief portions **524, 526, 536** are noted as having a concave configuration relative to the inner surface, and the portions outside of the concave configuration are generally convex relative to the inner surface. The relief portions **524, 526, 536,** by virtue of their concave configuration, provide desired flexibility to the patella pad **520** and mitigate pressure points between the patella pad **520** and a user's leg.

The patella pad **520** may define a grip pattern **532** that may be uniform or variable relative to the location of individual grip elements **533.** For example, the grip elements **533** may have a small size about the inner periphery of the patella pad **520** and the within the concave reliefs, relative to areas outside of these areas where the grip patterns may be bigger along the convex portions of the patella pad **520.** The grip pattern and elements **532, 533** advantageously prevent undesired translation of the patella pad **520** along the skin of a user.

Fig. 8C exemplifies how a removable stay system **540** may be provided within the knitted structure of a panel **544** for an orthopedic device **500.** Specifically, the panel **544** may form a pocket **548** that can accommodate a removable stay **542** through a slit **546** providing access to the pocket **548.** The pocket **548** preferably has a profile that closely approximates the stay **542** so the stay remains securely and snugly maintained within the pocket **548.**

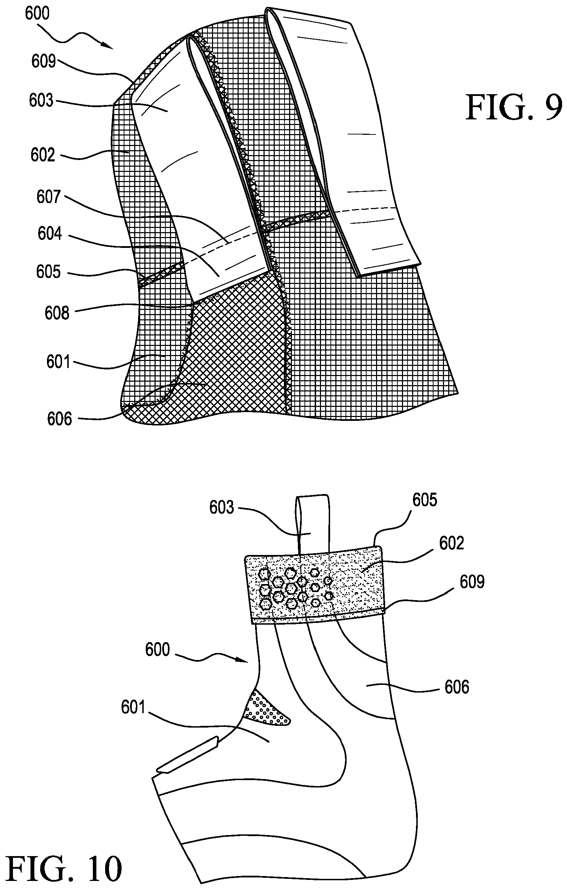

Turning now to Fig. 9, an elevational view of an embodiment of an orthopedic device is shown. In this particular embodiment, an ankle brace **600** is shown in an engaged configuration, comprising a main body panel arranged in this embodiment as a tubular body **601,** with an upper cuff **602** divided from the tubular body **601** by an upper folding portion **605.** An uppermost periphery of ankle brace **600** is defined by first edge **609.** Handles arranged in this embodiment as pull tabs **603** are attached to the ankle brace **600** at an attachment portion **604,** which may attach at a stay **606** similar to the foregoing embodiments and demarcated in this embodiment by a different fabric color and texture than surrounding portions of tubular body **601.** Pull tabs **603** may attach to the tubular body **601** by reinforcement stitching **607** and/or by adhesive **608.**

Fig. 10 depicts an elevational view of the embodiment of Fig. 9 in a disengaged configuration, depicting in particular how stay **606** may be arranged to extend through tubular body **601** in a particular configuration in order to advantageously apply pressure along critical portions of a limb, in this embodiment a region proximate to the Achilles tendon of a user. This structure has the added advantage of providing an improved path for the handles arranged in this embodiment as pull tabs **603** to exert forces on the ankle brace **600,** thus avoiding damage to the ankle brace **600** during donning and doffing.

In any of the aforementioned embodiments and variations thereof, an orthopedic device may include straps for added function to the device. The straps can be external, or they may be knitted as cables or laces into the brace, as discussed above, or they can be knitted as zones of low elasticity into the brace. For external straps, markers may be provided on the strap to guide the user how to apply the strap or adjust the cables for tensioning or reducing tension in the strap. The markers can be knitted into the knitting of the orthopedic device or otherwise applied, such as by heat transfer.

In another embodiment, straps or bands can be laminated into or over the orthopedic device. The orthopedic device may be knitted in a manner including zones of hook receivable material or structure, and the corresponding strap may include hook material for engaging the hook receivable zone. Loops may be formed directly by the orthopedic device for guiding the strap or cables.

It is possible to knit the orthopedic device in such a way that the active components are only visible on the inside, while the outside of the brace is more uniform in appearance. This enables the user to see how the brace functions before donning, while not being "too flashy" when worn.

While the disclosure discusses embodiments for the knee and ankle, orthopedic device embodiments of the disclosure may be used with other limbs, joints and anatomical portions including the torso, shoulder, elbow, wrist/hand, hip, knee, and foot/ankle.

## Claims

1. An orthopedic device (180), comprising:
a tubular sleeve (181), the tubular sleeve (181) having a main body defining an outer surface, and an upper cuff (182) extending from an upper end portion of the main body, the upper cuff (182) having inner and outer surfaces;
a frictional material (183) on an inner surface of the upper cuff (182);
**characterized in that** the upper cuff (182) is arranged to fold over the outer surface of the main body, such that the outer surface of the upper cuff (182) is adjacently against the outer surface of the main body,
wherein the upper cuff (182) has an upper band (193) having greater elasticity and reinforcement to apply greater circumferential pressure along a limb than the remainder of the upper cuff (182),
wherein the orthopedic device (180) further comprises a demarcation band (195) that is arranged to signal where the upper cuff (182) is to be folded, so that the upper cuff (182) and the frictional material (183) are completely disengaged from a user's leg when folded over,
wherein the demarcation band (195) has a different knitted structure from the upper cuff (182) to facilitate folding and demarcating the extent of the folding of the upper cuff (182) over the tubular sleeve (181).

2. The orthopedic device (180) of claim 1, further comprising a handle (187) secured to the main body, the handle extending outwardly from the main body in the disengaged configuration.

3. The orthopedic device (180) of claim 2, wherein the demarcation band (195) separates the upper cuff (182) and the tubular sleeve (181), the handle (187) extending from the demarcation band (195).

4. The orthopedic device (180) of claim 3, wherein the handle (187) extends from or proximate to the demarcation band (195).

5. The orthopedic device (180) of claim 3, wherein the demarcation band (195) facilitates location of the upper cuff (182) and folding of the upper cuff (182) over the main body at the demarcation band (195).

6. The orthopedic device (180) of claim 2, wherein the handle (187) comprises a loop.

7. The orthopedic device (180) of claim 2, wherein the handle (187) slidably secures to the main body.

8. The orthopedic device (180) of claim 1, wherein the main body defines the tubular sleeve (181) and the upper cuff (182) is generally circular in shape and forms an
extension of the tubular sleeve (181).

9. The orthopedic device (180) of claim 1, wherein the frictional material (183) is provided on the inner surface of the upper cuff (182) in a predetermined pattern.

10. The orthopedic device (180) of claim 9, wherein the predetermined pattern of the frictional material (183) defines a plurality of frictional elements (205) arranged about a circumference of the upper cuff (182).

11. The orthopedic device (180) of claim 9, wherein the plurality of frictional elements (205) comprises knitted silicone.

12. The orthopedic device (180) of claim 1, further comprising a hinge (408) connected to the main body, the hinge (408) having a segment terminating along the main body short of the upper cuff (182).

13. The orthopedic device (180) of claim 12, wherein the upper cuff (182) is adapted to fold over the segment of the hinge (408).

14. The orthopedic device (180) of claim 12, further comprising a hinge support (410) from which the hinge (408) secures and terminating along the main body short of the upper cuff (182).

15. The orthopedic device (180) of claim 14, further comprising a stay (184) supported by the hinge support (410).

16. The orthopedic device (180) of claim 14, wherein the main body defines a reinforcement band located coincident with the hinge support (410), the main body defines regions outside of the reinforcement band having a higher elasticity than the reinforcement band.

17. The orthopedic device (400) of claim 1, further comprising a least one strap extending from the main body and being located along the main body short of the upper cuff (182).

18. The orthopedic device (150) of claim 1, further comprising a lower cuff located at an opposite end of the main body than the upper cuff (182), the lower cuff provided with a frictional material (183) along an interior surface.

## Patentansprüche

1. Orthopädische Vorrichtung (180) mit:
einer Rohrhülse (181), wobei die Rohrhülse (181) aufweist einen Hauptkörper, der eine Außenfläche definiert, und eine obere Stulpe (182), die sich von einem oberen Endabschnitt des Hauptkörpers erstreckt, wobei die obere Stulpe (182) eine Innen- und eine Außenfläche aufweist;
ein Reibmaterial (183) auf einer Innenfläche der oberen Stulpe (182);
**dadurch gekennzeichnet, dass** die obere Stulpe (182) zum Falten über die Außenfläche des Hauptkörpers eingerichtet ist, sodass die Außenfläche der oberen Stulpe (182) an die Außenfläche des Hauptkörpers angrenzt,
wobei die obere Stulpe (182) ein oberes Band (193) aufweist, das eine größere Elastizität und Verstärkung aufweist, um größeren Umfangsdruck entlang einer Extremität als der Rest der oberen Stulpe (182) auszuüben,
wobei die orthopädische Vorrichtung (180) weiter ein Abgrenzungsband (195) aufweist, das eingerichtet ist, anzuzeigen, wo die obere Stulpe (182) zu falten ist, sodass die obere Stulpe (182) und das Reibmaterial (183) vollständig von einem Bein eines Anwenders entkoppelt ist, wenn es umgefaltet ist,
wobei das Abgrenzungsband (195) eine anders gestrickte Struktur als die obere Stulpe (182) aufweist, um Falten und Abgrenzen der Erstreckung der Faltung der oberen Stulpe (182) über die Rohrhülse (181) zu erleichtern.

2. Orthopädische Vorrichtung (180) gemäß Anspruch 1, weiter mit einer an den Hauptkörper befestigten Handhabe (187), wobei die Handhabe sich nach außen von dem Hauptkörper in der entkoppelten Konfiguration erstreckt.

3. Orthopädische Vorrichtung (180) gemäß Anspruch 2, wobei das Abgrenzungsband (195) die obere Stulpe (182) und die Rohrhülse (181) trennt, wobei die Handhabe (187) sich von dem Abgrenzungsband (195) erstreckt.

4. Orthopädische Vorrichtung (180) gemäß Anspruch 3, wobei die Handhabe (187) sich von oder benachbart zu dem Abgrenzungsband (195) erstreckt.

5. Orthopädische Vorrichtung (180) gemäß Anspruch 3, wobei das Abgrenzungsband (195) eine Lokalisierung der oberen Stulpe (182) und ein Falten der oberen Stulpe (182) über den Hauptkörper an dem Abgrenzungsband (195) erleichtert.

6. Orthopädische Vorrichtung (180) gemäß Anspruch 2, wobei die Handhabe (187) eine Schlaufe aufweist.

7. Orthopädische Vorrichtung (180) gemäß Anspruch 2, wobei die Handhabe (187) gleitend an dem Hauptkörper befestigt ist.

8. Orthopädische Vorrichtung (180) gemäß Anspruch 1, wobei der Hauptkörper die Rohrhülse (181) definiert und die obere Stulpe (182) hauptsächlich kreisförmig ist und eine Erweiterung der rohrförmigen Hülse (181) bildet.

9. Orthopädische Vorrichtung (180) gemäß Anspruch 1, wobei das Reibmaterial (183) auf der Innenfläche der oberen Stulpe (182) in einem vorbestimmten Muster vorgesehen ist.

10. Orthopädische Vorrichtung (180) gemäß Anspruch 9, wobei das vorbestimmte Muster des Reibmaterials (183) eine Vielzahl von Reibelementen (205) definiert, die um einen Umfang der oberen Stulpe (182) angeordnet sind.

11. Orthopädische Vorrichtung (180) gemäß Anspruch 9, wobei die Vielzahl von Reibelementen (205) gestricktes Silikon aufweist.

12. Orthopädische Vorrichtung (180) gemäß Anspruch 1, weiter mit einem Scharnier (408), das mit dem Hauptkörper verbunden ist, wobei das Scharnier (408) ein Segment aufweist, das entlang des Hauptkörpers kurz vor der oberen Stulpe (182) endet.

13. Orthopädische Vorrichtung (180) gemäß Anspruch 12, wobei die obere Stulpe (182) angepasst ist, um über das Segment des Scharniers (408) zu falten.

14. Orthopädische Vorrichtung (180) gemäß Anspruch 12, weiter mit einer Scharnierstütze (410), an der das Scharnier (408) befestigt ist und die entlang des Hauptkörpers kurz vor der oberen Stulpe (182) endet.

15. Orthopädische Vorrichtung (180) gemäß Anspruch 14, weiter mit einer Abstützung (184), die durch die Scharnierstütze (410) gestützt wird.

16. Orthopädische Vorrichtung (180) gemäß Anspruch 14, wobei der Hauptkörper ein Verstärkungsband definiert, das sich zusammenfallend mit der Scharnierstütze (410) befindet, wobei der Hauptkörper Regionen außerhalb des Verstärkungsbands definiert, die eine höhere Elastizität als das Verstärkungsband aufweisen.

17. Orthopädische Vorrichtung (400) gemäß Anspruch 1, weiter mit wenigstens einem Riemen, der sich von dem Hauptkörper erstreckt und sich entlang des Hauptkörpers kurz vor der oberen Stulpe (182) befindet.

18. Orthopädische Vorrichtung (150) gemäß Anspruch 1, weiter mit einer unteren Stulpe, die sich an einem gegenüberliegenden Ende des Hauptkörpers zu der oberen Stulpe (182) befindet, wobei die untere Stulpe mit einem Reibmaterial (183) entlang einer Innenfläche versehen ist.

## Revendications

1. Dispositif orthopédique (180), comprenant :
un manchon tubulaire (181), le manchon tubulaire (181) présentant un corps principal définissant une surface extérieure, et un parement supérieur (182) s'étendant d'une portion d'extrémité supérieure du corps principal, le parement supérieur (182) présentant des surfaces intérieure et extérieure ;
un matériau de friction (183) sur une surface intérieure du parement supérieur (182) ;
**caractérisé en ce que** le parement supérieur (182) est agencé pour se replier par-dessus la surface extérieure du corps principal, de telle sorte que la surface extérieure du parement extérieur (182) est située de manière adjacente contre la surface extérieure du corps principal,
dans lequel le parement supérieur (182) présente une bande supérieure (193) présentant une élasticité et un renfort plus élevés afin d'appliquer le long d'un membre une pression circonférentielle plus grande que sur le reste du parement supérieur (182) ;
dans lequel le dispositif orthopédique (180) comprend en outre une bande de démarcation (195), laquelle est agencée pour signaler où le parement supérieur (182) doit être plié, de sorte que le parement supérieur (182) et le matériau de friction (183) sont entièrement désolidarisés d'une jambe d'un utilisateur lorsque repliés, et
dans lequel la bande de démarcation (195) présente une structure tricotée différente de celle du parement supérieur (182) afin de faciliter le pliage et de démarquer l'étendue du pliage du parement supérieur (182) par-dessus le manchon tubulaire (181).

2. Dispositif orthopédique (180) selon la revendication 1, comprenant en outre une poignée (187) fixée sur le corps principal, la poignée s'étendant vers l'extérieur à partir du corps principal dans la configuration désolidarisée.

3. Dispositif orthopédique (180) selon la revendication 2, dans lequel la bande de démarcation (195) sépare le parement supérieur (182) et le manchon tubulaire (181), la poignée (187) s'étendant à partir de la bande de démarcation (195).

4. Dispositif orthopédique (180) selon la revendication 3, dans lequel la poignée (187) s'étend à partir de la bande de démarcation (195) et à proximité de celle-ci.

5. Dispositif orthopédique (180) selon la revendication 3, dans lequel la bande de démarcation (195) facilite la localisation du parement supérieur (182) et le repli du parement supérieur (182) par-dessus le corps principal sur la bande de démarcation (195).

6. Dispositif orthopédique (180) selon la revendication 2, dans lequel la poignée (187) comprend une boucle.

7. Dispositif orthopédique (180) selon la revendication 2, dans lequel la poignée (187) se fixe par coulissement sur le corps principal.

8. Dispositif orthopédique (180) selon la revendication 1, dans lequel le corps principal définit le manchon tubulaire (181), et le parement supérieur (182) présente une forme généralement circulaire et forme une extension du manchon tubulaire (181).

9. Dispositif orthopédique (180) selon la revendication 1, dans lequel le matériau de friction (183) est prévu sur la surface intérieure du parement supérieur (182) suivant un motif prédéterminé.

10. Dispositif orthopédique (180) selon la revendication 9, dans lequel le motif prédéterminé du matériau de friction (183) définit une pluralité d'éléments de friction (205) agencés autour d'une circonférence du parement supérieur (182).

11. Dispositif orthopédique (180) selon la revendication 9, dans lequel la pluralité d'éléments de friction (205) comprend de la silicone tricotée.

12. Dispositif orthopédique (180) selon la revendication 1, comprenant en outre une charnière (408) raccordée au corps principal, la charnière (408) présentant un segment se terminant le long du corps principal juste avant le parement supérieur (182).

13. Dispositif orthopédique (180) selon la revendication 12, dans lequel le parement supérieur (182) est apte à être replié par-dessus le segment de la charnière (408).

14. Dispositif orthopédique (180) selon la revendication 12, comprenant en outre un support de charnière (410) à partir duquel la charnière (408) se fixe et se terminant le long du corps principal juste avant le parement supérieur (182).

15. Dispositif orthopédique (180) selon la revendication 14, comprenant en outre un renfort (184) supporté par le support de charnière (410).

16. Dispositif orthopédique (180) selon la revendication 14, dans lequel le corps principal définit une bande de renfort située de manière à coïncider avec le support de charnière (410), et le corps principal définit des régions hors de la bande de renfort présentant une élasticité supérieure à celle de la bande de renfort.

17. Dispositif orthopédique (400) selon la revendication 1, comprenant en outre au moins une sangle s'étendant à partir du corps principal et située le long du corps principal juste avant le parement (182).

18. Dispositif orthopédique (150) selon la revendication 1, comprenant en outre un parement inférieur situé sur une extrémité du corps principal opposée à celle du parement supérieur (182), le parement inférieur étant doté d'un matériau de friction (183) le long d'une surface intérieure.
